# EUROPÄISCHE PATENTSCHRIFT

(11) **EP 0 686 692 B1**
(45) Veröffentlichungstag und Bekanntmachung des Hinweises auf die Patenterteilung: **28.08.2002**
(21) Anmeldenummer: 95104454.4
(22) Anmeldetag: 27.03.1995
(51) Int. Cl.: C11D 3/386

(54) **Verwendung alkalischer Proteasen in gewerblichen Textilwaschverfahren**
USE OF ALCALINE PROTEASES IN INDUSTRIAL TEXTIL WASHING PROCESSES
UTILISATION DES PROTEASES ALCALINES DANS LES PROCEDES INDUSTRIELS DE LAVAGE DE TEXTILES

(30) Priorität: 31.03.1994 DE 4411223
(43) Veröffentlichungstag der Anmeldung: 13.12.1995
(73) Patentinhaber: Genencor International GmbH, 31582 Nienburg/Weser (DE)
(72) Erfinder: Amory, Antoine, Dr., B-1330 Rixensart (BE); Clippe, André, B-1120 Brussels (BE); Konieczny-Janda, Gerhard, D-30982 Pattensen (DE)
(74) Vertreter: Kiddle, Simon John

(56) Entgegenhaltungen:
- EP-A- 0 415 296
- EP-A- 0 503 346
- DE-A- 4 219 104
- DATABASE WPI Section Ch, Week 9329 Derwent Publications Ltd., London, GB; Class D13, AN 93-231494 XP002027423 & JP 05 153 976 A (NIPPON KAGAKU KIKAI KOGYO KK) , 22.Juni 1993

## Beschreibung

Die Erfindung betrifft die Verwendung alkalischer Proteasen in gewerblichen Textilwaschverfahren sowie in diesen Verfahren verwendete Zusammensetzungen, welche alkalische Proteasen enthalten.

Die in gewerblichen Wäschereien eingesetzten Textilwaschmittel unterscheiden sich in vielfältiger Weise von den üblicherweise im Haushalt eingesetzten Waschmitteln. So werden in gewerblichen Wäschereien taktabhängig oder kontinuierlich arbeitende Großwaschanlagen mit einem sehr hohen Wäschedurchsatz eingesetzt, wobei bei den unterschiedlichen Waschstufen wie Benetzen, Vorwaschen, Klarwaschen und Spülen, unterschiedliche Waschmittelkombinationen je nach Textilart und Grad der Verschmutzung in die Flotte eindosiert werden. Auch die rationelle Ausnutzung von Wasser und Energie hat die Entwicklung besonderer teilkonfektionierter Waschmittelkombinationen für die gewerbliche Textilwäscherei erforderlich gemacht, die je nach Art und Verschmutzung der zu waschenden Textilien optimal auf die jeweilige Waschstufe abgestimmt werden können.

Der Einsatz proteasehaltiger Waschmittelzusammensetzungen in gewerblichen Wäschereien, z. B. zur Reinigung von mit Blut verunreinigter Krankenhauswäsche oder Schutzbekleidung aus fleischverarbeitenden Betrieben, ist bereits seit längerem bekannt. Auf Grund der in gewerblichen Textilwaschverfahren im Vergleich zu Haushaltswaschmitteln drastischeren Bedingungen werden an die hierbei verwendeten Proteasen besonders hohe Anforderungen gestellt. Neben einer guten Beständigkeit und Aktivität bei hochalkalischen pH-Werten sollten die Proteasen so über eine hohe Temperaturstabilität verfügen, um bei niedriger Konzentration für eine möglichst lange Zeitdauer bei den in gewerblichen Textilwaschverfahren hohen Temperaturen gute Waschergebnisse für den jeweiligen Waschgang zu erbringen. Außerdem sollten die eingesetzten alkalischen Proteasen möglichst unempfindlich gegen die in gewerblichen Textilwaschverfahren üblichen Waschmittelinhaltsstoffen wie z. B. Tenside, Bleichmittel oder desinfizierend wirksamen Bestandteile sein. Daher besteht immer noch Bedarf nach weiteren für gewerbliche Textilwaschverfahren geeigneten alkalischen Proteasen.

Es bestand daher die Aufgabe, neue für die Verwendung in gewerblichen Textilwaschverfahren geeignete alkalische Proteasen anzugeben.

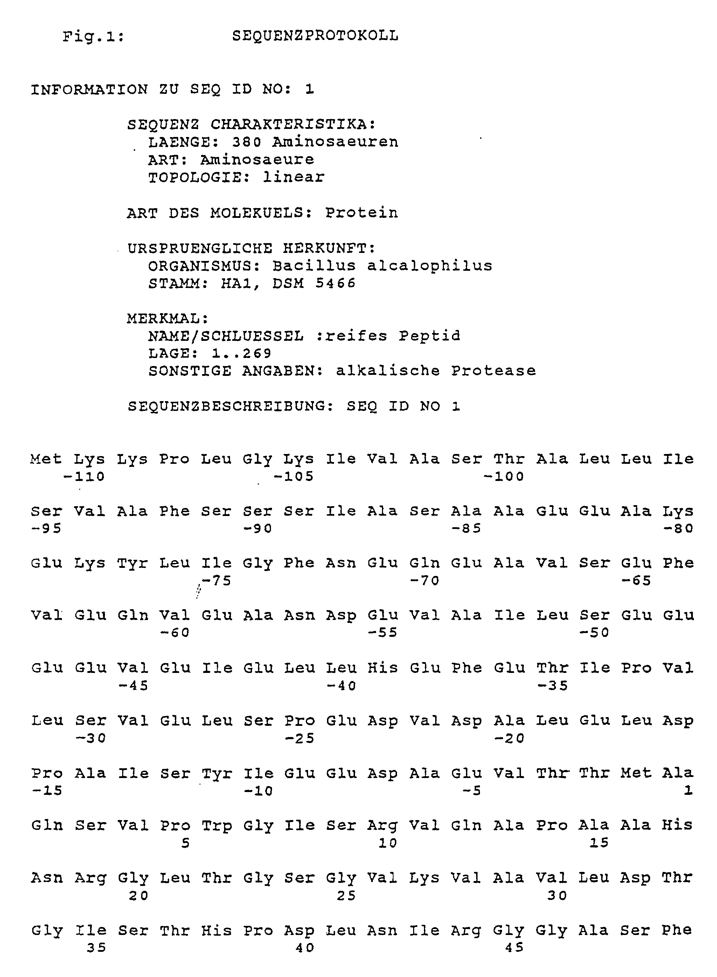

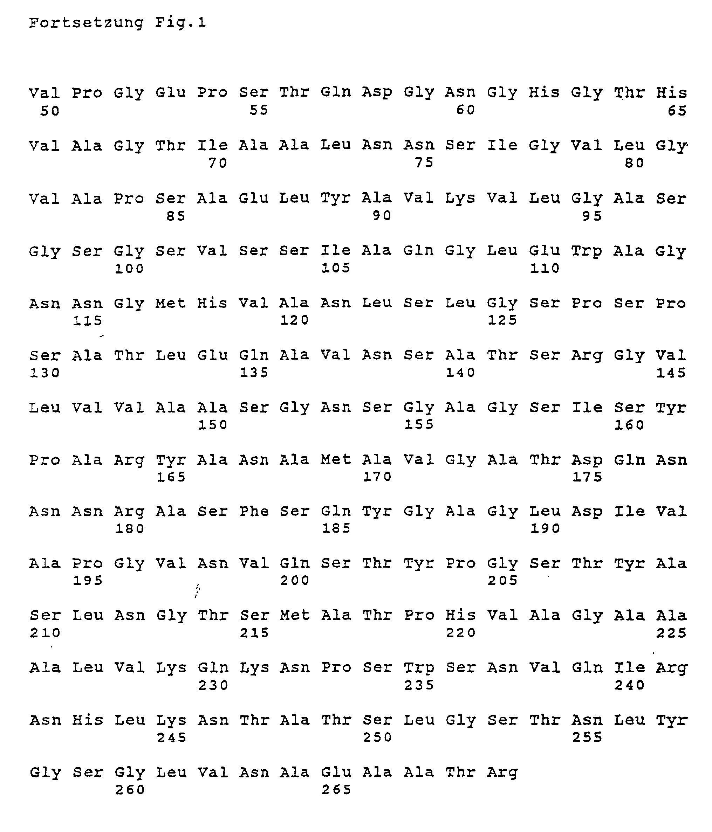

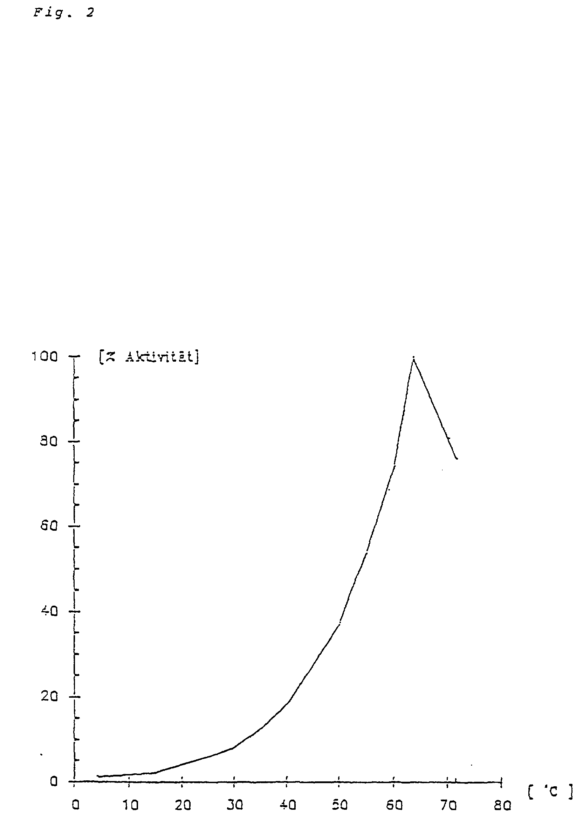

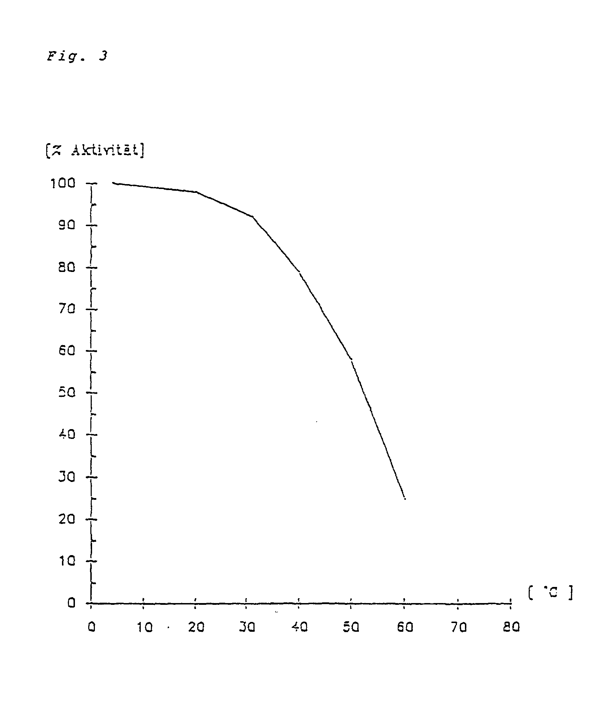

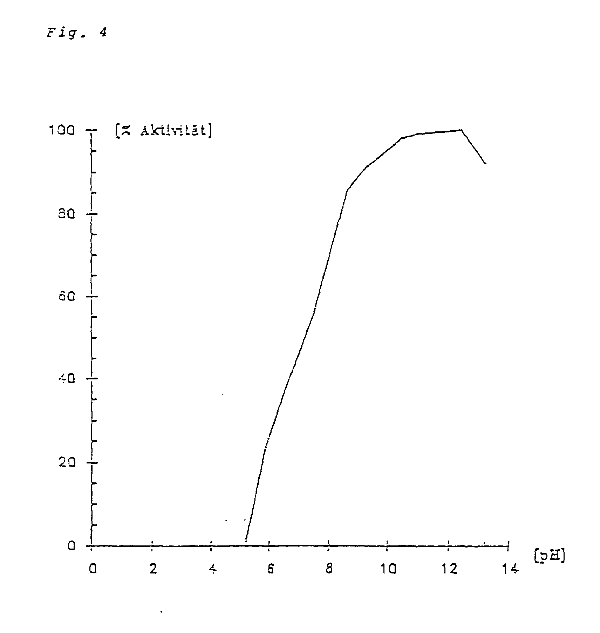

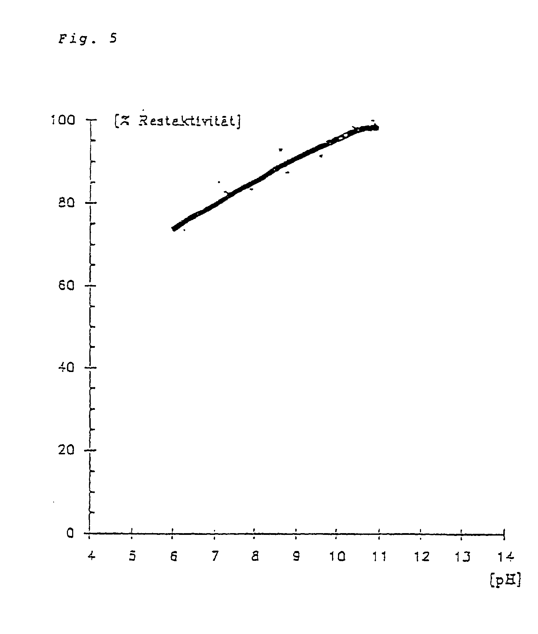

Es wurde nun gefunden, daß sich die nachstehend angegebenen alkalischen Bacillus-Proteasen mit hoher Waschwirksamkeit in gewerblichen Textilwaschverfahren einsetzen lassen. Gegenstand der Erfindung ist daher die Verwendung alkalischer Proteasen in Zusammensetzungen für gewerbliche Textilwaschverfahren, wobei man mindestens eine alkalische Protease ausgewählt aus der Gruppe alkalischer Bacillus-Proteasen aus
dem Bacillus-Stamm DSM 5466 mit einer Aminosäurensequenz, welche sich von der in Fig. 1 angegebenen Aminosäurensequenz durch wenigstens einen der Aminosäurenaustausche Q12R, N74R, M216Q, N237P, T249R unterscheidet,
verwendet.

Die genannten alkalischen Bacillus-Proteasen besitzen Molekulargewichte im Bereich von 26.000 bis 28.000 g/mol, gemessen durch SDS-Polyacrylamid-Gelelektrophorese gegenüber Referenzproteinen mit bekanntem Molekulargewicht. Ihr pH-Optimum liegt im Bereich von 8 bis 13,0, wobei unter pH-Optimum derjenige pH-Bereich verstanden wird, in dem die Proteasen maximale proteolytische Aktivität aufweisen. Auch weisen die genannten alkalischen Bacillus-Proteasen eine gute pH-Stabilität auf.

Eine alkalische Bacillus-Protease aus dem am 28.07.1989 unter der DSM-Nr. 5466 hinterlegten Bacillus-Stamm, welche eine Aminosäurensequenz besitzt, die sich von der in Fig. 1 angegebenen Aminosäurensequenz in den genannten Positionen unterscheidet, ist auf an sich bekannte Weise durch Punktmutation in der Aminosäurensequenz erhältlich, wie es z. B. in der EP-A-415 296 beschrieben wird.

In einer bevorzugten Variante verwendet man eine alkalische Bacillus-Protease aus dem Stamm DSM 5466 mit einer Aminosäurensequenz, welche sich von der in Fig. 1 angegebenen Aminosäurensequenz durch wenigstens einen der Aminosäurenaustausche Q12R, M216Q, N237P oder T249R, vorzugsweise durch die Aminosäurenaustausche N42R/N114R/M216Q unterscheidet. Die Zahlenangaben beziehen sich dabei auf die Position in der Aminosäurensequenz. Die Aminosäuren werden durch den Einbuchstabencode bezeichnet, wobei die ursprüngliche Aminosäure der Positionsangabe vorangestellt und die eingeführte Aminosäure der Positionsangabe nachgestellt ist. Diese Aminosäurenaustausche können auf an sich bekannte Weise durch Punktmutation in der Aminosäurensequenz erhalten werden, wie es z. B. in der EP-A-415 296 beschrieben ist.

Die genannten alkalischen Bacillus-Proteasen zeigen ungewöhnlich gute Waschleistung unter den in gewerblichen Wäschereien üblichen Bedingungen wie hohen pH-Werten, kurzen Waschzeiten und hohen Waschtemperaturen. Auch zeigen die genannten Proteasen eine überraschend hohe Beständigkeit gegen die in gewerblichen Textilwaschverfahren gebräuchlichen Waschmittelbestandteile. Erfindungsgemäß können die genannten alkalischen Bacillus-Proteasen vorteilhaft in gewerblichen Großraumtrommelwaschmaschinen oder vollkontinuierlich oder taktabhängig arbeitenden Gegenstrom-Waschstraßen verwendet werden. Besonders vorteilhaft werden dabei die alkalischen Bacillus-Proteasen bei sogenannten Mehrlaugenverfahren, z. B. Zweibadverfahren aus Vorwasch- und Klarwaschgang, der Flotte im Vorwaschgang zugegeben. Die Vorwäsche kann dabei bei den gewerblichen Textilwaschverfahren üblichen Bedingungen, z. B. bei Temperaturen von 30 bis 70 °C auf an sich bekannte Weise mit den üblicherweise in diesem Waschgang verwendeten Waschmittelinhaltsstoffen durchgeführt werden. Bei stark proteinhaltigen Verunreinigungen, z. B. stark blutbefleckter Wäsche aus Krankenhäusern, Großküchen oder fleischverarbeitenden Betrieben können die genannten Proteasen gegebenenfalls in einem dem Vorwaschgang vorgeschalteten Vorspülgang mit klarem kalten oder rückgeführtem warmen Wasser und den ansonsten hierfür üblichen Waschmittelinhaltsstoffen mit gutem Erfolg verwendet werden. Natürlich können die genannten alkalischen Bacillus-Proteasen auch in allen anderen, gewerblichen Textilwaschverfahren, z. B. in auf bestimmte Textil- und Verschmutzungsarten abgestimmten gewerbliche Textilwaschverfahren, wie z. B. bei der desinfizierenden Wäsche von Textilien aus dem Krankenhaus-Sektor, erfindungsgemäß verwendet werden.

Weiterhin umfaßt die Erfindung Zusammensetzungen für gewerbliche Textilwaschverfahren, welche mindestens eine alkalische Bacillus-Protease aus
dem Bacillus-Stamm DSM 5466 mit einer Aminosäurensequenz, welche sich von der in Fig. 1 angegebenen Aminosäurensequenz in mindestens einer der Positionen Q12R, N74R, M216Q, N237P, T249R unterscheidet,
enthalten.

In einer bevorzugten Variante enthalten die erfindungsgemäßen Zusammensetzungen eine alkalische Bacillus-Protease aus dem Stamm DSM 5466 mit einer Aminosäurensequenz, welche sich von der in Fig. 1 angegebenen Aminosäurensequenz durch wenigstens einen der Aminosäurenaustausche Q12R, M216Q, N237P, T249R, insbesondere durch die Aminosäurenaustausche N42R/N114R/M-216Q unterscheidet.

Vorzugsweise sollten in den erfindungsgemäßen Zusammensetzungen solche alkalischen Bacillus-Proteasen eingesetzt werden, welche eine Enzymaktivität von 50.000 bis 1.000.000 DU je Gramm Enzympräparat aufweisen. Unter "DU" wird dabei die enzymatische Aktivität in Delft Units verstanden, wobei 1.000 DU der proteolytischen Aktivität entsprechen, die bei einem Volumen von 1 ml einer 2 %(W/W)igen Enzymlösung nach Abbau von Casein eine Extinktionsdifferenz (1 cm Lichtweg; 275 nm; Bestimmung gegen Blindprobentest) von 0,4000 ergibt. Die genannten alkalischen Bacillus-Proteasen können dabei in den für gewerbliche Textilwaschverfahren üblichen Formulierungen einzeln oder gewünschtenfalls auch in Kombination miteinander, gegebenenfalls auch in Kombination mit anderen auf den gewerblichen Sektor gebräuchlichen Waschmittelproteasen oder anderen an sich üblichen Waschmittelenzymen, wie z. B. Amylasen, Lipasen, Cellulasen, Pektinasen, Nukleasen, Oxidoreduktasen etc., eingesetzt werden. Bezogen auf die Trockensubstanz der Gesamtzubereitung sollte der Anteil der genannten Bacillus-Proteasen in den erfindungsgemäßen Waschmittelformulierungen bevorzugt bei 0,1 bis 5 Gew.-%, insbesondere bei 0,2 bis 2,0 Gew.-% liegen.

Die erfindungsgemäßen Zusammensetzungen können in Form der für gewerbliche Textilwaschverfahren gebräuchlichen Vollwaschmittel, Alleinwaschmittel sowie Vorwasch- oder Vorspülmittel vorliegen. Je nach Waschmitteltyp können dabei alle an sich im Stand der Technik üblichen Waschmittelinhaltsstoffe wie Tenside, Bleichmittel, Buildersubstanzen (Gerüststoffe), Waschhilfschemikalien, optische Aufheller sowie weitere übliche Komponenten wie z. B. Soda, Metasilikat, Orthophosphat oder Natrium-Triphosphat in an sich üblichen Mengen enthalten. Zu den möglichen Waschmittelinhaltsstoffen gehören weiterhin z. B. Verstärker, Enzymstabilisatoren, Schmutzträger und/oder Kompatibilisierungsmittel, Komplex- und Chelatbildner, Seifenschaumregulatoren und Zusatzstoffe wie Korrosionsinhibitoren, Antielektrostatika, Duftstoffe, Desinfektionsmittel, Bleichmittelaktivatoren, Persäurebleichmittelvorstufen und Vergrauungsinhibitoren.

Bevorzugt sind die erfindungsgemäßen Waschmittelzusammensetzungen Vorwaschmittel, wie sie bei gewerblichen Textilwaschverfahren im Temperaturbereich von 30 bis 70 °C in sogenannten Mehrlaugenverfahren, z. B. in Zweibadverfahren aus Vorwasch- und Klarwaschgang, eingesetzt werden. Neben den genannten alkalischen Bacillus-Proteasen können die erfindungsgemäßen Vorwaschmittel alle hierfür auf dem gewerblichen Sektor üblichen Inhaltsstoffe wie z. B. nichtionische Tenside, Phosphate, Carbonate, Silikate, gegebenenfalls Perborate und/oder Bleichaktivatoren, Vergrauungsinhibitoren, Polycarboxylate, optische Aufheller sowie gegebenenfalls weitere Puffer und Hilfsstoffe enthalten. Es können auch kommerziell erhältliche Waschmittelformulierungen eingesetzt werden, denen zusätzlich die genannten alkalischen Bacillus-Proteasen in den angegebenen Mengen zugesetzt wurden. Falls erforderlich, können diese kommerziell erhältlichen Formulierungen dabei auch Bleichmittel auf Sauerstoffbasis enthalten. Beispiele für derartige kommerziell erhältliche Waschmittelformulierungen für den gewerblichen Sektor sind TEN-COLOR^{R} oder TENAX CONC.^{R}.

Die erfindungsgemäßen Waschmittelzusammensetzungen können auf an sich bekannte Weise in Pulverform, z. B. in Form von Granulaten, Prills oder Pellets, gegebenenfalls auch mit Oberflächenüberzügen versehen, konfektioniert sein. Auf Grund ihrer guten Stabilität lassen sich die genannten Bacillus-Proteasen auch in Flüssigformulierungen einsetzen.

Bei den für gewerbliche Textilwaschverfahren üblichen Bedingungen wie hochalkalischen pH-Werten, z. B. pH-Werten über 11,0 sowie hohen Waschtemperaturen von bis zu 70 °C zeigen die genannten alkalischen Bacillus-Proteasen überraschend gute Wascheigenschaften. Dies ist umso überraschender, als im Vergleich zur üblichen Haushaltswäsche durch die in gewerblichen Wäschereien eingesetzten oft vollkontinuierlich arbeitenden Gegenstromwaschanlagen zumeist nur relativ kurze Waschzeiten zur Verfügung stehen. Neben einer hohen Temperaturbeständigkeit zeigen die genannten alkalischen Bacillus-Proteasen dabei eine hohe Enzymstabilität in Gegenwart der in gewerblichen Waschmitteln gebräuchlichen Inhaltsstoffe. Auch gegen die im gewerblichen Sektor gebräuchlichen Bleichmittel, wie sie z. B. in gewerblichen Desinfektions-Waschmitteln für den Krankenhaus-Sektor eingesetzt werden, insbesondere gegen Sauerstoffbleichkonzentrate z. B. auf der Basis von Perborat oder Wasserstoffperoxid, sind die genannten alkalischen Bacillus-Proteasen bei erfindungsgemäßer Verwendung stabil.

Die folgenden Beispiele sollen die Erfindung erläutern, ohne sie jedoch in ihrem Umfang zu beschränken.

### Erläuterungen zu den Figuren:

### Figur 1:

### Sequenzprotokoll der Aminosäurensequenz (SEQ ID NO1) der alkalischen Protease aus Bacillus alcalophilus HA1 (DSM 5466)

### Beispiele

Die Sequenzierung der in Fig. 1 angegebenen Aminosäurensequenz der alkalischen Protease aus Bacillus alcalophilus HA1 (DSM 5466) über Ermittlung der entsprechenden Nukleotidsequenz ist in den Beispielen 1 bis 4 der EP-A2-415 296 beschrieben.

Unter der Nummer DSM 5466 ist der als Bacillus alcalophilus HA1 benannte Bacillus alcalophilus-Stamm bei der Deutschen Sammlung von Mikroorganismen (DSM) am 28.07.1989 hinterlegt worden.

### Beispiel 1:

### Herstellung von durch Mutation in der Aminosäurensequenz variierter alkalischer Proteasen

Die Herstellung alkalischer Proteasen, welche sich von der in Fig. 1 angegebenen Aminosäurensequenz der alkalischen Protease aus Bacillus alcalophilus HA1 (DSM 5466) durch wenigstens einen der Aminosäurenaustausche Q12R, N42R, N74R, N114R, N115R, Q135R, M216Q, N237P, T249R unterscheiden, wurde auf an sich bekannte Weise durch gerichtete Mutagenese in DNA-Teilsequenzen des entsprechenden Proteasegens durchgeführt. Mit den Zahlenangaben ist dabei die entsprechende Position in der in Fig. 1 angegebenen Aminosäurensequenz bezeichnet, wobei der Positionsangabe im an sich bekannten Einbuchstabencode die ursprüngliche Aminosäure vorangestellt und die eingeführte Aminosäure der Positionsangabe nachgestellt ist. Ausführlich wird für die angegebenen Mutationen das Verfahren der gerichteten Mutagenese in den Beispielen 5 bis 18 der EP 415 296 beschrieben. In bezug auf die Aminosäurenaustausche in den Positionen 42, 114, 216 und 249 sei zusätzlich noch auf die DE 43 04 161 verwiesen.

### Prinzipiell umfaßte das Verfahren die folgenden an sich bekannten Verfahrensschritte:

Aus dem Naturisolat Bacillus alcalophilus HA1 (DSM 5466) wurde nach der Methode von Saito et al. (1963, Biochim. Biophys. Acta 72, 619-629) chromosomale DNA isoliert und mit der Restriktionsendonuklease Sau3A partiell hydrolysiert. Die Restriktionsfragmente wurden durch Elektrophorese aufgetrennt und die Fragmente mit einer Größe von 3 bis 8 Kilobasen isoliert. Die isolierten und größenselektierten DNA-Fragmente aus Bacillus alcalophilus HA1 wurden mit Vektor-DNA des an sich bekannten Plasmids pUB 110 in vitro auf an sich bekannte Weise neu kombiniert. Mit der erhaltenen in vitro neu kombinierten DNA wurden Protoplasten des Stammes Bacillus subtilis BD224 (Bacillus Genetic Stock Center 1A46) nach der von S. Chang und N. Cohen (1979, Mol. Gen. Genet. 168, 111-115) beschriebenen Methode transformiert. Die Transformanten wurden auf Platten mit Neomycin selektiert. Aus einem Klon wurde die Plasmid-DNA nach dem Handbuch von Maniatis et al. (Maniatis et al. = T. Maniatis, E. F. Fritsch, J. Sambrook, Molecular Cloning, A Laboratory Manual, Cold Spring Harbor Laboratory, 1982) isoliert. Das in diesem Plasmid enthaltende Fragment aus der B. alcalophilus-DNA hatte eine Größe von 4,1 KB und enthielt die vollständige DNA-Sequenz für die hochalkalische Protease aus Bacillus alcalophilus HA1 (DSM 5466) (vgl. Beispiel 1 und 2 der EP 415 296).

Das die vollständige DNA-Sequenz für die hochalkalische Protease aus Bacillus alcalophilus HA1 (DSM 5466) enthaltende Plasmid wurde mit Aval geschnitten. Die überstehenden Enden wurden auf an sich bekannte Weise (Maniatis et al., S. 114) zum DNA-Doppelstrang aufgefüllt. Nach anschließender Restriktion dieser DNA mit XbaI wurde das N-terminale 1.618 BP umfassende Fragment isoliert und auf an sich bekannte Weise in den Vektor pBS kloniert. Der resultierende Vektor enthielt das N-terminale Ende der für die in Fig. 1 abgebildete Aminosäurensequenz codierenden DNA (vgl. Beispiel 5 der EP 415 296).

In analoger Weise wurde ein Vektor erstellt, der ein 658 BP umfassendes für das C-terminales Ende der entsprechenden Protease codierendes DNA-Fragment enthielt. Hierfür wurde das die vollständige DNA-Sequenz enthaltende Plasmid mit den Restriktionsendonukleasen XbaI und Asp718 geschnitten und in die entsprechende Schnittstelle des bekannten Vektors pBS kloniert (vgl. Beispiel 7 der EP 415 296).

Die gerichteten Mutationen wurden in der das C-terminale oder das N-terminale Ende enthaltenden DNA-Teilsequenz mit der von Kunkel, T. A. (1985, Proc. Natl. Acad. Sci. USA 82, 488-492) beschriebenen "primer extension" Technik durchgeführt. Hierzu wurden die entsprechenden Vektoren zunächst in ihre uracilierten einzelsträngigen Analoga auf an sich bekannte Weise umgewandelt, indem mit einem der beiden Vektoren transformierte E. coli CJ236-Bakterien kultiviert wurden, welche zusätzlich mit dem Helfer-Phagen M13 K07 (bezogen von Bio-Rad Laboratories, Richmond, Kalifornien) infiziert wurden. Das Bakterium E. coli CJ236 ist einem an sich bekannten Uracil-N-Glycosylase-Mangelmutante, welche bei der Replikation der Vektoren statt Thymidin das Nukleotid Uracil in die DNA-Sequenz des Vektors einbaut. Uracilierte Vektoren lassen sich auf an sich bekannte Weise vorteilhaft für in vitro-Reaktionen der gerichteten Mutagenese einsetzen, da nach Beendigung der Reaktionen der Uracil-haltige DNA-Einzelstrang, der als Matritze zur Erzeugung mutierter DNA-Stränge diente, durch Behandlung mit Uracil-N-Glycosylase beseitigt werden kann. Der Einsatz der genannten Helfer-Phagen war für die Synthese der Hüllproteine für die gebildete uracilierte einzelsträngige Vektor-DNA nötig. Umhüllte uracilierte Einzelstrang-Vektor-DNA wurde aus dem transformierten Wirtsorganismus E. coli CJ236 ausgeschleust und anschließend aus dem Kulturmedium isoliert.

Die isolierten, uracilierten DNA-Einzelstrang-Vektoren des C-terminalen bzw. N-terminalen Endes wurden mit synthetischen Oligonukleotiden hybridisiert, welche eine Mutationsstelle enthielten und gleichzeitig als Primer für die nachfolgende Ergänzung zum vollständigen DNA-Doppelstrang mit Mutation dienten. Die hierbei eingesetzten synthetischen Oligonukleotide wurden auf an sich bekannte Weise nach der Methode von Beaucage, S. L. und Caruthers, M. H. (1981, Tetrahedron Letters 22, 1859-1862) hergestellt. Die Synthese des zweiten DNA-Stranges wurde auf an sich bekannte Weise mit Hilfe von T4-DNA-Polymerase und nachfolgender Ligation mit T4-DNA-Ligase durchgeführt (Kunkel et al., 1987, Methods in Enzymol. 154, 367-382). Die gebildete Doppelstrang-Vektor-DNA wurde in E. coli MC 1061 transformiert und die mutierten Vektoren wurden durch Überprüfen der entsprechenden unitären Restriktionsendonukleasen-Erkennungsstellen, die mit den synthetischen Oligonukleotiden eingeführt bzw. entfernt wurden, identifiziert.

Zur Herstellung von z. B. zwei Mutationen entweder im N-terminalen oder im C-terminalen Teil der Protease-DNA wurde das Verfahren nach Einführung einer ersten Mutation in analoger Weise unter Verwendung eines weiteren synthetischen Oligonukleotides zur Einführung einer zweiten Mutation wiederholt.

Es wurden Expressionsvektoren mit Mutationen im C-terminalen Teil bzw. N-terminalen Teil der Protease-DNA-Sequenz hergestellt, indem die durch die gerichtete Mutagenese erhaltenen DNA-Sequenzen mit Restriktionsendonukleasen geschnitten wurden und mit einer Vektor-DNA, welche den entsprechenden anderen terminalen Teil der DNA-Sequenz sowie alle für die Expression notwendigen Elemente enthielt, ligiert wurden. Die erhaltenen Vektoren stellten vollständige Expressionsvektoren mit geeignetem Leseraster zur Expression der entsprechend mutierten Protease dar. Ausführlich ist die Herstellung dieser Expressionsvektoren in Beispiel 16 der EP 415 296 beschrieben. Für die folgenden Mutationen wurden Expressionsvektoren hergestellt:

| | |
|---|---|
| DSM 5466 Mut. | N114R/M216Q |
| DSM 5466 Mut. | N115R/Q135R* |
| DSM 5466 Mut. | N42R/N114R/M216Q |
| DSM 5466 Mut. | N42R/N114Q/N115Q* |
| DSM 5466 Mut. | N114R/N237P |
| DSM 5466 Mut. | N42R/N114R* |
| DSM 5466 Mut. | Q12R/N42R/N114R |
| DSM 5466 Mut. | N114R/N237P/T249R |
| DSM 5466 Mut. | Q12R/N42R/N114R |

| | |
|---|---|
| [* als Vergleich] | |

Die mutierten hochalkalischen Proteasen wurden hergestellt, indem B. subtilis BD 224 mit jeweils einen der obengenannten Expressionsvektoren auf an sich bekannte Weise transformiert und kultiviert wurden. Aus den Kulturüberständen der transformierten und kultivierten Stämme wurden die mutierten hochalkalischen Proteasen nach bekannten Verfahren isoliert. Ausführliche Angaben zur Isolierung der mutierten Proteasen finden sich in den Beispielen 16 und 18 der EP 415 296.

Die durch Mutation in der Aminosäurensequenz variierten alkalischen Proteasen auf Grundlage des Bacillus alcalophilus HA1 (DSM 5466) wurden in den in Beispiel 3 beschriebenen Waschversuchen eingesetzt.

### Beispiel 2:

### Waschversuche unter in gewerblichen Textilwaschverfahren üblichen Bedingungen

Es wurden Waschversuche mit angeschmutztem Testgewebe unter in gewerblichen Verfahren üblichen Bedingungen durchgeführt. Als Testgewebe wurde ein mit Blut, Milch und Tusche angeschmutztes Polyester-Baumwoll-Mischgewebe (EMPA117, bezogen von der eidgenössischen Materialprüfungsanstalt, St. Gallen, Schweiz) ein mit Eigelb und Tusche angeschmutztes Polyester-Baumwoll-Mischgewebe (EY-PC, eigene Herstellung) sowie ein mit Milch und Tusche angeschmutztes Polyester-Baumwoll-Mischgewebe (M-PC, eigene Herstellung) eingesetzt. Gewaschen wurde in Laborwaschmaschinen vom Typ Zelltex Polycolor, wobei als Waschmittelbasisformulierungen die im gewerblichen Sektor üblichen unter den Handelsnamen TEN COLOR^{R} und TENAX CONC.^{R} (Hersteller Firma J. P. Haas/Steinau) erhältlichen Vorwaschmittel verwendet wurden. Gewaschen wurde im Temperaturbereich von 15 °C bis 60 °C für 45 Minuten (2 °C/min; 22,5 min Haltezeit bei 60 °C) oder im Temperaturbereich von 15 °C bis 65 °C für 25 Minuten (5 °C/min; 15 min Haltezeit bei 65 °C). Die Wasserhärte lag bei 15° dH; die Enzymkonzentration betrug 0,71 mg reine Protease pro Liter Waschlösung. Die enzymhaltige Waschmittellösung wirkte in einem rotierenden Probengefäßkammer, das über ein Wasserbad entsprechend dem Temperaturprogramm gesteuert wurde, auf das Testge-webe ein. Nach dem Waschvorgang wurde das Testgewebe zweimal mit entsalztem Wasser gespült und anschließend gebügelt.

Die Waschleistung der Proteasen wurde durch Messung der Reflexion des gewaschenen Testgewebes mit Hilfe eines Remissionsphotometers bestimmt. Ebenfalls wurde die Reflexion des nur mit der Waschmittelbasisformulierung gewaschenen Testgewebes ermittelt. Die Differenz dieser beiden Reflexionswerte ist als sogenannter Delta R-Wert ein Maß für die Waschleistung der jeweiligen Protease. Zum Vergleich mit Proteasen des Standes der Technik wurden alle Waschversuche ebenfalls unter identischen Bedingungen für die kommerziell unter dem Handelsnamen Opticlean^{R} erhältliche Protease durchgeführt.

Tabelle 1 zeigt die Waschleistungen der erfindungsgemäß verwendeten Proteasen mit einer bleichmittelfreien Waschmittelformulierung für gewerbliche Textilwaschverfahren, wie sie unter dem Handelsnamen TENAX CONC. kommerziell erhältlich ist.

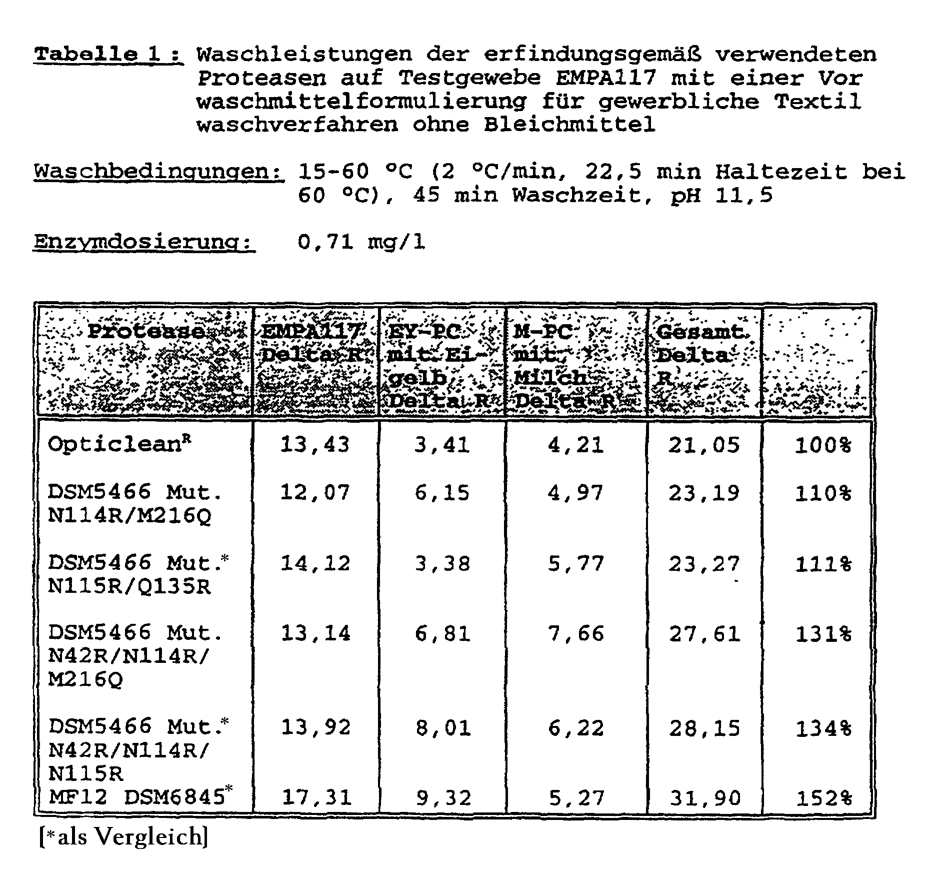

Tabelle 2 zeigt die Waschleistungen der erfindungsgemäß verwendeten Proteasen mit einer unter dem Handelsnamen TEN-COLOR^{R} kommerziell erhältlichen Waschmittelformulierung für gewerbliche Textilwaschverfahren, welche Bleichmittel auf Sauerstoffbasis (Perborat) enthält.

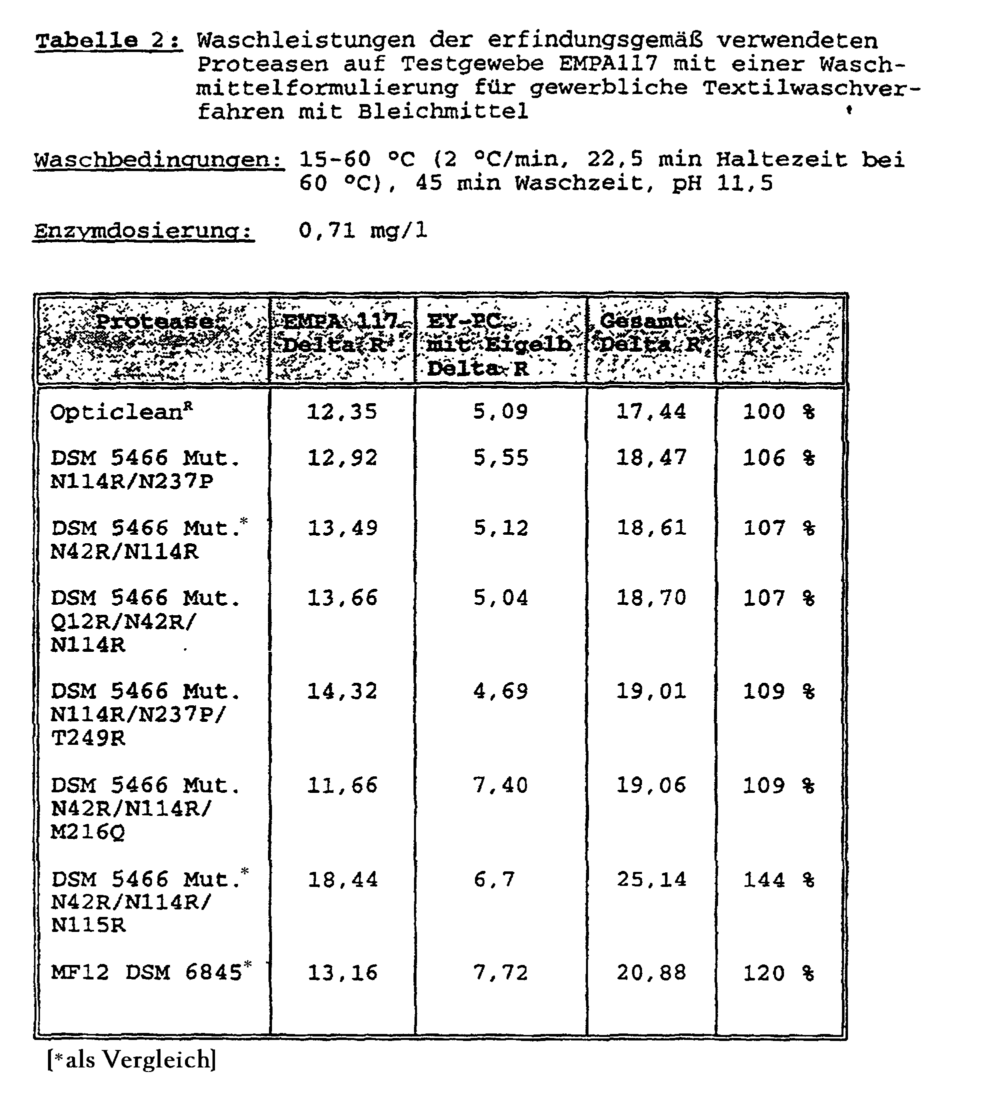

Die hohen Reflexionswerte der erfindungsgemäß verwendeten Proteasen belegen deren hohe Waschleistungen auf proteinverschmutztes Gewebe unter den in gewerblichen Textilwaschverfahren üblichen Bedingungen (hochalkalische pH-Werte, lange Haltezeiten bei hohen Temperaturen).

Zusätzlich neben der Waschwirksamkeit auf das Testgewebe EM-PA117 wurden auch noch Waschversuche mit dem mit Eigelb-Tusche angeschmutzten Testgewebe EY-PC und dem mit Milch-Tusche angeschmutzten Gewebe M-PC durchgeführt. Außerdem wurden die Waschbedingungen auf eine Aufheizrate von 5 °C/min und eine Haltezeit von 15 min bei 65 °C verschärft. Auch bei diesen Waschversuchen wurde die bleichmittelhaltige Waschmittelformulierung TEN-COLOR^{R} für gewerbliche Textilwaschverfahren eingesetzt. Tabelle 3 zeigt die dabei erhaltenen Ergebnisse.

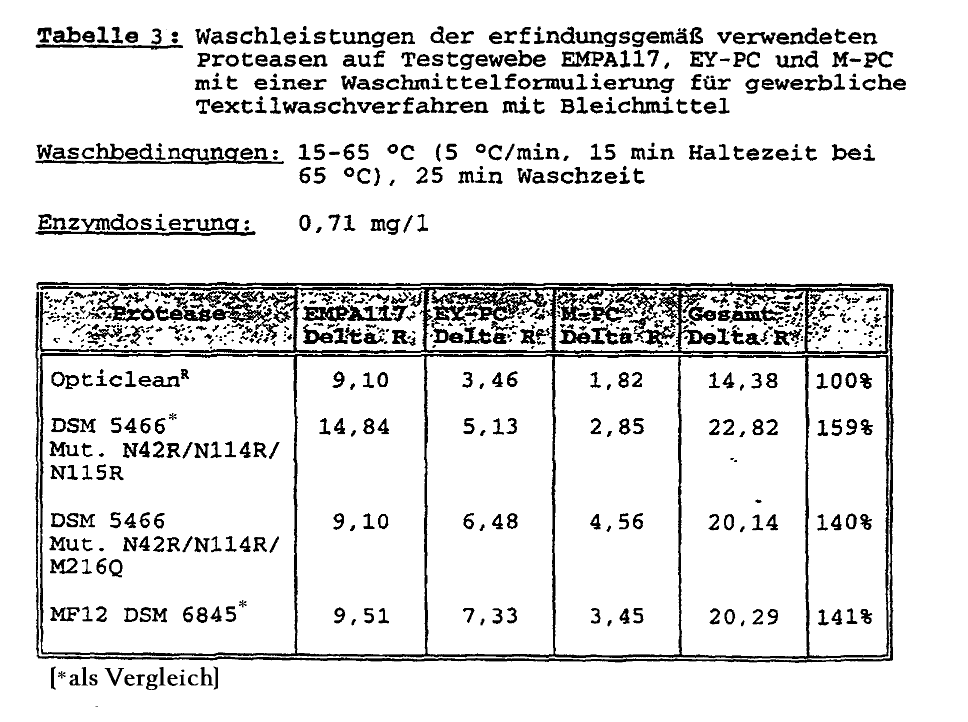

Aus Tabelle 1 bis 3 wird deutlich, daß die erfindungsgemäß verwendeten Proteasen sehr hohe Waschleistungen auf unterschiedlichen Gewebearten zeigen, die mit unterschiedlichen Proteinverunreinigungen angeschmutzt sind. Beeinträchtigungen der Enzymstabilität durch das hochalkalische Millieu der Waschlauge, die hohe Waschtemperatur sowie durch das Bleichmittel sind dabei praktisch nicht feststellbar. Bei der kurzen Waschzeit von nur 25 min werden dabei außergewöhnlich gute Waschleistungen festgestellt. Diese Ergebnisse zeigen damit die besonders gute Eignung der genannten Proteasen für gewerbliche Textilwaschverfahren.

## Patentansprüche

1. Verwendung alkalischer Proteasen in Zusammensetzungen für gewerbliche Textilwaschverfahren, **dadurch gekennzeichnet, daß** man mindestens eine alkalische Protease ausgewählt aus der Gruppe alkalischer Bacillus-Proteasen aus
dem Bacillus-Stamm DSM 5466 mit einer Aminosäurensequenz, welche sich von der in Fig. 1 angegebenen Aminosäurensequenz durch wenigstens einen der Aminosäurenaustausche Q12R, N74R, M216Q, N237P, T249R unterscheidet,
verwendet.

2. Verwendung alkalischer Proteasen nach Anspruch 1, **dadurch gekennzeichnet, daß** man eine alkalische Bacillus-Protease aus dem Stamm DSM 5466 mit einer Aminosäurensequenz, welche sich von der in Fig. 1 angegebenen Aminosäurensequenz durch wenigstens einen der Aminosäurenaustausche Q12R, N74R, M216Q, N237P, T249R unterscheidet, verwendet.

3. Verwendung alkalischer Proteasen nach Anspruch 2, **dadurch gekennzeichnet, daß** man eine alkalische Bacillus-Protease aus dem Stamm DSM 5466 mit einer Aminosäurensequenz, welche sich von der in Fig. 1 angegebenen Aminosäurensequenz durch die Aminosäurenaustausche N42R/N-114R/M216Q unterscheidet, verwendet.

4. Zusammensetzungen für gewerbliche Textilwaschverfahren, **dadurch gekennzeichnet, daß** sie mindestens eine alkalische Protease ausgewählt aus der Gruppe alkalischer Bacillus-Proteasen
aus dem Bacillus-Stamm DSM 5466 mit einer Aminosäurensequenz, welche sich von der in Fig. 1 angegebenen Aminosäurensequenz durch wenigstens einen der Aminosäurenaustausche Q12R, N74R, M216Q, N237P, T249R unterscheidet,
enthalten.

5. Zusammensetzungen nach Anspruch 4, **dadurch gekennzeichnet, daß** sie eine alkalische Bacillus-Protease aus dem Stamm DSM 5466 mit einer Aminosäurensequenz, welche sich von der in Fig. 1 angegebenen Aminosäurensequenz durch wenigstens einen der Aminosäurenaustausche Q12R, N74R, M216Q, N237P, T249R unterscheidet, enthalten.

6. Zusammensetzungen nach Anspruch 5, **dadurch gekennzeichnet, daß** sie eine alkalische Bacillus-Protease aus dem Stamm DSM 5466 mit einer Aminosäurensequenz, welche sich von der in Fig. 1 angegebenen Aminosäurensequenz durch die Aminosäurenaustausche N42R/N114R/M216Q unterscheidet, enthalten.

7. Zusammensetzungen nach einem der Ansprüche 4 bis 6, **dadurch gekennzeichnet, daß** sie für Mehrlaugenverfahren, insbesondere Zweibadverfahren, geeignete Vorwaschmittel sind.

8. Zusammensetzungen nach einem der Ansprüche 4 bis 7, **dadurch gekennzeichnet, daß** sie alkalische Bacillus-Proteasen mit einer Aktivität von 50.000 bis 1.000.000 DU je Gramm Enzympräparat enthalten.

9. Zusammensetzungen nach einem der Ansprüch 8, **dadurch gekennzeichnet, daß** sie alkalische Bacillus-Proteasen in einer Menge von 0,1 bis 5,0 Gew.-% enthalten.

10. Zusammensetzungen nach einem der Ansprüche 4 bis 9, **dadurch gekennzeichnet, daß** sie Sauerstoffbleichmittel enthalten.

## Claims

1. Use of alkaline proteases in compositions for industrial textile washing processes, **characterized in that** at least one alkaline protease selected from the group of alkaline bacillus-proteases of the bacillus strain DSM 5466 with an amino-acid sequence which differs from the amino-acid sequence indicated in Fig. 1 by at least one of the amino-acid substitutions Q12R, N74R, M216Q, N237P, T249R, is used.

2. Use of alkaline proteases according to Claim 1, **characterized in that** an alkaline bacillus-protease of the strain DSM 5466 with an amino-acid sequence which differs from the amino-acid sequence indicated in Fig. 1 by at least one of the amino-acid substitutions Q12R, N74R, M216Q, N237P, T249R, is used.

3. Use of alkaline proteases according to Claim 2, **characterized in that** an alkaline bacillus-protease of the strain DSM 5466 with an amino-acid sequence which differs from the amino-acid sequence indicated in Fig. 1 by the amino-acid substitutions N42R/N114R/M216Q, is used.

4. Compositions for industrial textile washing processes, **characterized in that** they contain at least one alkaline protease selected from the group of alkaline bacillus-proteases of the bacillus strain DSM 5466 with an amino-acid sequence which differs from the amino-acid sequence indicated in Fig. 1 by at least one of the amino-acid substitutions Q12R, N74R, M216Q, N237P, T249R.

5. Compositions according to Claim 4, **characterized in that** they contain an alkaline bacillus-protease of the strain DSM 5466 with an amino-acid sequence which differs from the amino-acid sequence indicated in Fig. 1 by at least one of the amino-acid substitutions Q12R, N74R, M216Q, N237P, T249R.

6. Compositions according to Claim 5, **characterized in that** they contain an alkaline bacillus-protease of the strain DSM 5466 with an amino-acid sequence which differs from the amino-acid sequence indicated in Fig. 1 by the amino-acid substitutions N42R/N114R/M216Q.

7. Compositions according to any one of Claims 4 to 6, **characterized in that** they are prewash agents suitable for multistage washing processes, in particular two-bath processes.

8. Compositions according to any one of Claims 4 to 7, **characterized in that** they contain alkaline bacillus-proteases with an activity of 50,000 to 1,000,000 DU per gram of enzyme preparation.

9. Compositions according to any one of Claims [*sic*] 8, **characterized in that** they contain alkaline bacillus-proteases in a proportion of 0.1 to 5.0 %wt.

10. Compositions according to any one of Claims 4 to 9, **characterized in that** they contain oxygen bleaching agents.

## Revendications

1. Utilisation de protéases alcalines dans des compositions pour des procédés industriels de lavage de textiles **caractérisée en ce qu'**on utilise au moins une protéase alcaline choisie dans le groupe de protéases alcalines de bacilles formé de
la souche du Bacille DSM 5466 avec une séquence d'acides aminés qui se différencie de la séquence d'acides aminés donnée à la figure 1 par au moins l'un des échanges d'acides aminés Q12R, N74R, M216Q, M237P, T249R.

2. Utilisation de protéases alcalines selon la revendication 1, **caractérisée en ce qu'**on utilise une protéase alcaline de Bacille de la souche DSM 5466 avec une séquence d'acides aminés qui se différencie de la séquence d'acides aminés donnée à la figure 1 par au moins l'un des échanges d'acides aminés Q12R, N74R, M216Q, M237P, T249R.

3. Utilisation de protéases alcalines selon la revendication 2, **caractérisée en ce qu'**on utilise une protéase alcaline de Bacille de la souche DSM 5466 avec une séquence d'acides aminés qui se différencie de la séquence d'acides aminés donnée à la figure 1 par les échanges d'acides aminés N42R/N114R/M216Q.

4. Compositions pour procédés industriels de lavage de textiles **caractérisées en ce qu'**elles contiennent au moins une protéase alcaline choisie dans le groupe de protéases alcalines de bacille
formé de la souche de Bacille DSM 5466 avec une séquence d'acides aminés qui se différencie de la séquence d'acides aminés donnée à la figure 1 par au moins l'un des échanges d'acides aminés Q12R, N74R, M216Q, N237P, T249R.

5. Compositions selon la revendication 4, **caractérisées en ce qu'**elles contiennent une protéase alcaline de Bacille de la souche DSM 5466 avec une séquence d'acides aminés qui se différencie de la séquence d'acides aminés donnée à la figure 1 par au moins l'un des échanges d'acides aminés Q12R, N74R, M216Q, N237P, T249R.

6. Compositions selon la revendication 5, **caractérisées en ce qu'**elles contiennent une protéase alcaline de bacille de la souche DSM 5466 avec une séquence d'acides aminés qui se différencie de la séquence d'acides aminés indiquée à la Figure 1 par les échanges d'acides aminés N42R, N114R, M216Q.

7. Compositions selon l'une quelconque des revendications 4 à 6, **caractérisées en ce qu'**elles sont un agent de prélavage approprié pour des procédés à plusieurs lessives, en particulier des procédés à deux bains.

8. Compositions selon l'une quelconque des revendications 4 à 7, **caractérisées en ce qu'**elles contiennent des protéases alcalines de bacille avec une activité de 50 000 à 1 000 000 UD par gramme de préparation d'enzyme.

9. Compositions selon la revendication 8, **caractérisées en ce qu'**elles contiennent des protéases alcalines de bacille en une quantité de 0,1 à 5,0% en poids.

10. Compositions selon l'une quelconque des revendications 4 à 9 **caractérisées en ce qu'**elles contiennent un agent blanchissant oxygéné.
